(19) Europäisches Patentamt / European Patent Office / Office européen des brevets

(11) **EP 4 365 850 A1**

(12) **EUROPEAN PATENT APPLICATION**
published in accordance with Art. 153(4) EPC

(43) Date of publication:
**08.05.2024 Bulletin 2024/19**

(21) Application number: **21965891.1**

(22) Date of filing: **30.11.2021**

(51) International Patent Classification (IPC):
*G06T 17/00* (2006.01)

(52) Cooperative Patent Classification (CPC):
**G06T 17/00**

(86) International application number:
**PCT/CN2021/134421**

(87) International publication number:
**WO 2023/097447 (08.06.2023 Gazette 2023/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Shenzhen Shokz Co., Ltd.**
**Shenzhen, Guangdong 518108 (CN)**

(72) Inventors:
• **LI, Meiqi**
**Shenzhen, Guangdong 518000 (CN)**
• **SU, Lei**
**Shenzhen, Guangdong 518000 (CN)**
• **ZHOU, Xin**
**Shenzhen, Guangdong 518000 (CN)**
• **LIAO, Fengyun**
**Shenzhen, Guangdong 518000 (CN)**
• **QI, Xin**
**Shenzhen, Guangdong 518000 (CN)**

(74) Representative: **Fuchs Patentanwälte**
**Partnerschaft mbB**
**Tower 185**
**Friedrich-Ebert-Anlage 35-37**
**60327 Frankfurt am Main (DE)**

(54) **MOVEMENT DATA CALIBRATION METHOD AND SYSTEM**

(57) The present application provides a method and system for motion data calibration, which can convert motion data during a movement of a user from three-dimensional posture data of a coordinate system whose three axes are mutually perpendicular to each other, to two-dimensional data under a target coordinate system, such that the movement of the user may be divided to a movement in a horizontal direction and a movement in a vertical direction. The method and system are capable of calibrating motion data without requiring the user to perform a calibration action.

3000

Obtain action data during a movement of a user, the action data comprises at least one posture signal corresponded to at least one measurement position on the user, each of the posture signal of the at least one posture signal comprises three-dimensional posture data under the original coordinate corresponded to the measurement position — S3020

Establish a target coordinate system — S3040

Convert each of the posture signal to two-dimensional posture data in the target coordinate system — S3060

Determine relative motion between the at least one measurement position based on the two-dimensional posture data corresponded to each of the posture signal — S3080

FIG. 6

EP 4 365 850 A1

**Description**

**TECHNICAL FIELD**

This application relates to the field of wearable device technologies, and in particular, to a motion data calibration method and system.

**BACKGROUND**

As people pay more attention to scientific exercise and physical health, motion monitoring devices are under tremendous development. Currently, a motion monitoring device mainly monitors action parameters during a movement of a user with a sensor. In the existing technologies, when the motion monitoring device performs coordinate calibration with the sensor, the user is required to perform a series of calibration actions (for example, raising two arms forward or raising two arms laterally), so that the motion monitoring device can convert posture data in a sensor coordinate system to posture data in a human body coordinate system based on the calibration actions. Whenever the user adjusts a position of the motion monitoring device, coordinate calibration needs to be performed again; otherwise, a calculation result may be affected to cause poor user experience.

Therefore, it is necessary to provide a motion data calibration method and system that can calibrate motion data without requiring a user to perform a calibration action.

**SUMMARY**

This application provides a motion data calibration method and system that can calibrate motion data without requiring a user to perform a calibration action.

In a first aspect, the present application provides a method for calibrating motion data, comprising: obtaining action data during a movement of a user, wherein the action data includes at least one posture signal corresponding to at least one measurement position on a body of the user, and each posture signal of the at least one posture signal includes three-dimensional posture data of a corresponded measurement position in an original coordinate system; establishing a target coordinate system, wherein the target coordinate system includes an X-axis, a Y-axis, and a Z-axis mutually perpendicular to each other; and converting each posture signal to two-dimensional posture data in the target coordinate system.

In some embodiments, each posture signal includes data obtained by a posture sensor, and the original coordinate system includes a coordinate system in which the posture sensor is located.

In some embodiments, the posture sensor includes at least one of an acceleration sensor, an angular velocity sensor, or a magnetic sensor.

In some embodiments, each posture signal includes data obtained by an image sensor, and the original coordinate system includes a coordinate system in which the image sensor is located.

In some embodiments, the three-dimensional posture data includes angle data and angular velocity data on the X-axis, the Y-axis and the Z-axis mutually perpendicular to each other.

In some embodiments, the converting of each posture signal to the two-dimensional posture data in the target coordinate system includes: obtaining a pre-stored conversion relationship between the target coordinate system and the original coordinate system; converting each posture signal to the three-dimensional motion data in the target coordinate system based on the conversion relationship, wherein the three-dimensional motion data includes at least angular velocity data on the X-axis, angular velocity data on the Y-axis, and angular velocity data on the Z-axis; and converting the three-dimensional motion data in the target coordinate system to the two-dimensional posture data in the target coordinate system.

In some embodiments, the Z-axis of the target coordinate system is in a direction opposite to a vertical direction of a gravity acceleration.

In some embodiments, the two-dimensional posture data in the target coordinate system includes: horizontal posture data, including horizontal angle data and horizontal angular velocity data during a movement of the user in a horizontal plane perpendicular to the Z-axis; and vertical posture data, including vertical angle data and vertical angular velocity data during a movement of the user in any vertical plane perpendicular to the horizontal plane.

In some embodiments, the converting of the three-dimensional motion data in the target coordinate system to the two-dimensional posture data in the target coordinate system includes: converting the angular velocity data on the X-axis and the angular velocity data on the Y-axis to vertical angular velocity data by using a vector law; performing time integration with the vertical angular velocity data based on a time corresponding to a start position and a time corresponding to an end position during the movement of the user, to obtain the vertical angle data; using the angular velocity data on the Z-axis as the horizontal angular velocity data; and performing time integration on the horizontal angular velocity data based on the time corresponding to the start position and the time corresponding to the end position during the movement of the user, to obtain the horizontal angle data.

In some embodiments, the method further includes determining relative motion between the at least one measurement position based on the two-dimensional posture data corresponding to each posture signal.

In a second aspect, the present application provides a motion data calibration system, includes: at least one storage medium storing at least one instruction set for calibrating motion data; and at least one processor in communication with the at least one storage medium, where when the motion data calibration system runs, the at least one processor reads the at least one instruction set and implements the motion data calibration method according to the first aspect.

As can be learned from the foregoing technical solutions, in the motion data calibration method and system provided in this application, the action data during the movement of the user can be converted from the three-dimensional posture data on the X-axis, the Y-axis, and the Z-axis mutually perpendicular to each other to the two-dimensional data in the target coordinate system, that is, posture data in the horizontal plane and posture data in the vertical plane. In this way, actions during movement of the user are classified to movement in a horizontal direction and movement in a vertical direction, and a data discrepancy caused by different orientations of the user is avoided. The method and system can eliminate influence of the orientations of the user on the motion data. Therefore, the method and system can calibrate the motion data without requiring the user to perform any calibration action.

## BRIEF DESCRIPTION OF DRAWINGS

This application is further described by using exemplary embodiments. The exemplary embodiments are described in detail with reference to accompanying drawings. The embodiments are non-restrictive. In the embodiments, same numerals represent same structures.

FIG. 1 is a schematic diagram of an application scenario of a motion monitoring system according to some embodiments of this application;
FIG. 2 is an exemplary schematic diagram of hardware and/or software of a wearable device according to some embodiments of this application;
FIG. 3 is an exemplary schematic diagram of hardware and/or software of a computing device according to some embodiments of this application;
FIG. 4 is an exemplary structural diagram of a wearable device according to some embodiments of this application;
FIG. 5 is an exemplary flowchart of a motion monitoring method according to some embodiments of this application;
FIG. 6 is an exemplary flowchart of a motion data calibration method according to some embodiments of this application;
FIG. 7 is a schematic diagram of a target coordinate system according to some embodiments of this application;
FIG. 8 is an exemplary flowchart for converting to two-dimensional posture data according to some embodiments of this application; and
FIG. 9 is a diagram of a coordinate system during a movement of a user according to some embodiments of this application.

## DETAILED DESCRIPTION OF EMBODIMENTS

To describe the technical solutions in the embodiments of this application in a clearer way, the following briefly introduces the accompanying drawings required for describing the embodiments. Apparently, the accompanying drawings in the following description merely show some examples or embodiments of this application, and a person of ordinary skill in the art may further apply this application to other similar scenarios based on the accompanying drawings without creative efforts. Unless explicitly indicated from a linguistic context or specifically explained otherwise, same reference numbers in the figures represent same structures or operations.

It should be understood that the terms "system", "apparatus", "unit", and/or "module" used in this application are for the purpose of distinguishing between different components, elements, parts, portions, or assemblies at different levels. However, if other terms can achieve the same purpose, the terms may be replaced with other expressions.

As shown in this application or claims, unless explicitly indicated in an exceptional case in a context, the terms "a", "one", "one type of", and/or "the" do not necessarily refer to singular forms, but may also include plural forms. Generally, the terms "comprise" and "include" merely indicate that explicitly identified steps and elements are included, but the steps and elements do not constitute an exclusive list, and a method or device may also include other steps or elements.

In this application, a flowchart is used to describe operations performed by a system according to some embodiment of this application. It should be understood that previous or subsequent operations are not necessarily performed in a fixed order. Conversely, steps may be performed in a reverse order or processed simultaneously. In addition, other operations may also be added to the processes, or one or some of the operations may be removed from the processes.

This application provides a motion monitoring system. The motion monitoring system may obtain an action signal during a movement of a user, where the action signal includes at least a myoelectric signal, a posture signal, an electrocardio signal, a respiratory frequency signal, or the like. The system may monitor the action of the movement of the user based on at least feature information corresponding to the myoelectric signal or feature information corresponding to the posture signal. For example, by using frequency information and amplitude information corresponding to the myoelectric signal, and an angular velocity, a direction of the angular velocity, an angular velocity value of the angular velocity, an angle, displacement information, stress, and the like corresponding to the posture signal, the system determines an action type of the user, a quantity of actions, action quality, and an action time, or physiological parameter information or the like when the user implements the action. In some embodiments, the motion monitoring system may further generate feedback about an exercise action of the user based on a result of analysis of the exercise action of the user, to guide the user in exercise. For example, when the exercise action of the user does not meet the standard, the motion monitoring system may send out prompt information (for example, a voice prompt, a vibration prompt, or a current stimulation) to the user. The motion monitoring system may be applied to a wearable device (for example, a garment, a wristband, or a helmet), a medical test device (for example, a myoelectric tester), an exercise device, or the like. By obtaining the action signal during movement of the user, the motion monitoring system may accurately monitor the action of the user and provide feedback, without the involvement of any professional. Therefore, exercise costs of the user can be reduced while exercise efficiency of the user is improved.

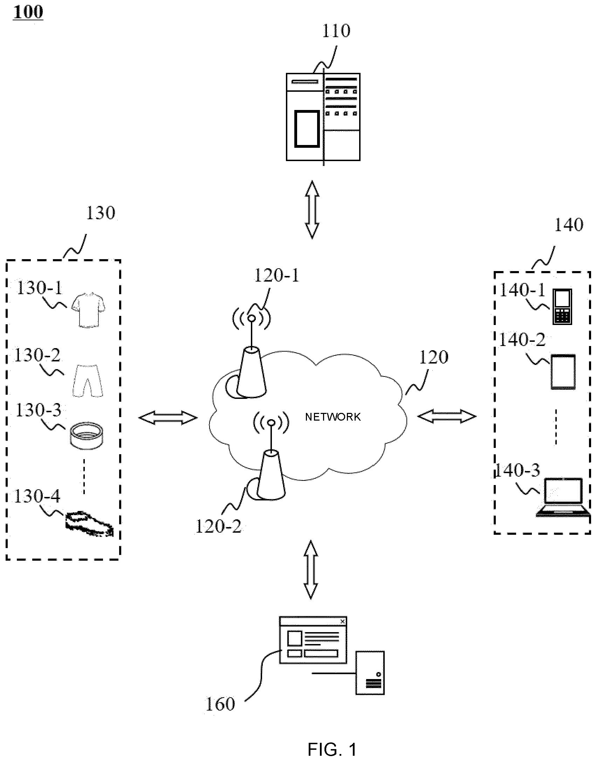

FIG. 1 is a schematic diagram of an application scenario of a motion monitoring system according to some embodiments of this application. As shown in FIG. 1, the motion monitoring system 100 may include a processing device 110, a network 120, a wearable device 130, and a mobile terminal device 140. In some embodiments, the motion monitoring system 100 may further include a motion data calibration system 180. The motion monitoring system 100 may obtain an action signal (for example, a myoelectric signal, a posture signal, an electrocardio signal, or a respiratory frequency signal) used to represent an action during a movement of a user, monitor the action of the movement of the user based on the action signal of the user, and provide feedback.

For example, the motion monitoring system 100 may monitor a exercise action of the user and provide feedback. When the user wearing the wearable device 130 performs exercises, the wearable device 130 may obtain the action signal of the user. The processing device 110 or the mobile terminal device 140 may receive and analyze the action signal of the user, to determine whether the exercise action of the user meets the standard and monitor the action of the user. Specifically, the monitoring of the action of the user may include determining an action type of the action, a quantity of actions, action quality, and an action time, or physiological parameter information or the like when the user implements the action. Further, the motion monitoring system 100 may generate feedback about the exercise action of the user based on a result of analysis of the exercise action of the user, so as to guide the user in exercise.

In another example, the motion monitoring system 100 may monitor a running action of the user and provide feedback. For example, when the user wearing the wearable device 130 takes running exercise, the motion monitoring system 100 may monitor whether the running action of the user meets the standard, and whether a running time conforms to a health standard. When the running time of the user is too long, or the running action is incorrect, the exercise device may provide feedback of a movement status of the user to the user to prompt the user to adjust the running action or the running time.

In some embodiments, the processing device 110 may be configured to process information and/or data related to movement of the user. For example, the processing device 110 may receive the action signal of the user (for example, the myoelectric signal, the posture signal, the electrocardio signal, or the respiratory frequency signal), and further extract feature information corresponding to the action signal (for example, feature information corresponding to the myoelectric signal in the action signal, or feature information corresponding to the posture signal). In some embodiments, the processing device 110 may perform specific signal processing, for example, signal segmentation or signal preprocessing (for example, signal correction processing or filtering processing), on the myoelectric signal or the posture signal captured by the wearable device 130. In some embodiments, the processing device 110 may also determine, based on the action signal of the user, whether the action of the user is correct. For example, the processing device 110 may determine, based on the feature information (for example, amplitude information or frequency information) corresponding to the myoelectric signal, whether the action of the user is correct. In another example, the processing device 110 may determine, based on the feature information (for example, an angular velocity, a direction of the angular velocity, an acceleration of the angular velocity, an angle, displacement information, and stress) corresponding to the posture signal, whether the action of the user is correct. In another example, the processing device 110 may determine, based on the feature information corresponding to the myoelectric signal and the feature information corresponding to the posture signal, whether the action of the user is correct. In some embodiments, the processing device 110 may further determine whether the physiological parameter information during movement of the user conforms to the health standard. In some embodiments, the processing device 110 may further send a corresponding instruction for feeding back the movement status of the user. For example, when the user takes running exercise, the motion monitoring system 100 detects that the user has run for a too long time period. In this case, the processing device 110 may send an instruction to the mobile terminal device 140 to prompt the user to adjust the running time. It should be noted that the feature information corresponding to the posture signal is not limited to the foregoing angular velocity, direction of the angular velocity, acceleration of the angular velocity, angle, displacement information, stress, and the like, and may also be other feature information. Any parameter information that can reflect relative motion of the body of the user may be the feature information corresponding to the posture signal. For example, when a posture sensor is a strain sensor, a bending angle and a bending direction at the user's joint may be obtained by measuring a magnitude of resistance that varies with a tensile length in the strain sensor.

In some embodiments, the processing device 110 may be a local or remote device. For example, through the network 120, the processing device 110 may access information and/or data stored in the wearable device 130 and/or the mobile terminal device 140. In some embodiments, the processing device 110 may be directly connected to the wearable device 130 and/or the mobile terminal device 140 to access information and/or data stored therein. For example, the processing device 110 may be located in the wearable device 130, and may exchange information with the mobile terminal device 140 through the network 120. In another example, the processing device 110 may be located in the mobile terminal device 140, and may exchange information with the wearable device 130 through the network. In some embodiments, the processing device 110 is executable on a cloud platform.

In some embodiments, the processing device 110 may process data and/or information related to motion monitoring to perform one or more of functions described in this application. In some embodiments, the processing device 110 may obtain the action signal captured by the wearable device 130 during movement of the user. In some embodiments, the processing device 110 may send a control instruction to the wearable device 130 or the mobile terminal device 140. The control instruction may control a switch status of the wearable device 130 and each sensor of the wearable device 130, and may further control the mobile terminal device 140 to send out prompt information. In some embodiments, the processing device 110 may include one or more sub-processing devices (for example, single-core processing devices or multi-core processing devices).

The network 120 may facilitate the exchange of data and/or information in the motion monitoring system 100. In some embodiments, one or more components of the motion monitoring system 100 may send data and/or information to other components of the motion monitoring system 100 through the network 120. For example, the action signal captured by the wearable device 130 may be transmitted to the processing device 110 through the network 120. In another example, a confirmation result about the action signal in the processing device 110 may be transmitted to the mobile terminal device 140 through the network 120. In some embodiments, the network 120 may be any type of wired or wireless network.

The wearable device 130 refers to a garment or device having a donning function. In some embodiments, the wearable device 130 may include, but is not limited, to an upper garment apparatus 130-1, a pant apparatus 130-2, a wristband apparatus 130-3, a shoe apparatus 130-4, and the like. In some embodiments, the wearable device 130 may include a plurality of sensors. The sensors may obtain various action signals (for example, the myoelectric signal, the posture signal, temperature information, a heart rate, and the electro cardio signal) during movement of the user. In some embodiments, the sensor may include, but is not limited to, one or more of a myoelectric sensor, a posture sensor, a temperature sensor, a humidity sensor, an electrocardio sensor, a blood oxygen saturation sensor, a Hall sensor, a galvanic skin sensor, a rotation sensor, and the like. For example, the myoelectric sensor may be disposed at a position of a muscle (for example, a biceps brachii, a triceps brachii, a latissimus dorsi, or a trapezius) of a human body in the upper garment apparatus 130-1, and the myoelectric sensor may fit onto the user's skin and capture the myoelectric signal during movement of the user. In another example, an electrocardio sensor may be disposed near a left pectoral muscle of the human body in the upper garment apparatus 130-1, and the electrocardio sensor may capture the electrocardio signal of the user. In another example, the posture sensor may be disposed at a position of a muscle (for example, a gluteus maximus, a vastus lateralis, a vastus medialis, or a gastrocnemius) of the human body in the pant apparatus 130-2, and the posture sensor may capture the posture signal of the user. In some embodiments, the wearable device 130 may further provide feedback about the action of the user. For example, during movement of the user, when an action of a part of the body does not conform to the standard, a myoelectric sensor corresponding to the part may generate a stimulation signal (for example, a current stimulation or striking signal) to alert the user.

It should be noted that the wearable device 130 is not limited to the upper garment apparatus 130-1, the pant apparatus 130-2, the wristband apparatus 130-3, and the shoe apparatus 130-4 shown in FIG. 1, but may further include any other device for motion monitoring, such as a helmet apparatus or a kneepad apparatus. This is not limited herein. Any device that can use the motion monitoring method included in this application shall fall within the protection scope of this application.

In some embodiments, the mobile terminal device 140 may obtain information or data in the motion monitoring system 100. In some embodiments, the mobile terminal device 140 may receive processed motion data from the processing device 110, and feedback a motion record or the like based on the processed motion data. Exemplary feedback manners may include, but are not limited to, a voice prompt, an image prompt, a video presentation, a text prompt, and the like. In some embodiments, the user may obtain an action record during movement of the user by using the mobile terminal device 140. For example, the mobile terminal device 140 may be connected to the wearable device 130 through the network 120 (for example, a wired connection or a wireless connection), and the user may obtain the action record during movement of the user by using the mobile terminal device 140, where the action record may be transmitted to the processing device 110 through the mobile terminal device 140. In some embodiments, the mobile terminal device 140 may include one or any combination of a mobile apparatus 140-1, a tablet computer 140-2, a notebook computer 140-3, and the like. In some embodiments, the mobile apparatus 140-1 may include a mobile phone, a smart home apparatus, a smart mobile apparatus, a virtual reality apparatus, an augmented reality apparatus, or the like, or any combination thereof. In some embodiments, the smart home apparatus may include a smart appliance control apparatus, a smart monitoring apparatus, a smart television, a smart camera, or the like, or any combination thereof. In some embodiments, the smart mobile apparatus may include a smartphone, a personal digital assistant (PDA), a game apparatus, a navigation apparatus, a POS apparatus, or the like, or any combination thereof. In some embodiments, the virtual reality apparatus and/or the augmented reality apparatus may include a virtual reality helmet, virtual reality glasses, virtual reality eye masks, an augmented reality helmet, augmented reality glasses, augmented reality eye masks, or the like, or any combination thereof.

In some embodiments, the motion monitoring system 100 may further include a motion data calibration system 180. The motion data calibration system 180 may be used to process action data related to user motion and can perform the motion data calibration method described in this specification. Specifically, the motion data calibration system 180 may receive the action data during movement of the user, and can convert the action data from the three-dimensional posture data on the X-axis, the Y-axis, and the Z-axis mutually perpendicular to each other to the two-dimensional posture data in the target coordinate system, that is, posture data in the horizontal plane and posture data in the vertical plane. In this way, actions during movement of the user are classified into movement in a horizontal direction and movement in a vertical direction, and a data discrepancy caused by different orientations of the user is avoided. The motion data calibration system 180 can eliminate adverse impact of the orientations of the user on the motion data. Therefore, the motion data may be calibrated without requiring the user to perform any calibration action. The action data may be three-dimensional posture data of a measurement position on a body of the user during movement. The action data and the motion data calibration method are described in detail later. In some embodiments, the motion data calibration system 180 may be integrated with the processing device 110. In some embodiments, the motion data calibration system 180 may alternatively be integrated with the mobile terminal device 140. In some embodiments, the motion data calibration system 180 may alternatively exist independently of the processing device 110 and the mobile terminal device 140. The motion data calibration system 180 may be in communication with the processing device 110, the wearable device 130, and the mobile terminal device 140 to transmit and exchange information and/or data. In some embodiments, through the network 120, the motion data calibration system 180 may access information and/or data stored in the processing device 110, the wearable device 130, and/or the mobile terminal device 140. In some embodiments, the wearable device 130 may be directly connect to the processing device 110 and/or the mobile terminal device 140 to access information and/or data stored therein. For example, the motion data calibration system 180 may be located in the processing device 110, and may exchange information with the wearable device 130 and the mobile terminal device 140 through the network 120. In another example, the motion data calibration system 180 may be located in the mobile terminal device 140, and may exchange information with the processing device 110 and the wearable device 130 through the network. In some embodiments, the motion data calibration system 180 is executable on the cloud platform, and may exchange information with the processing device 110, the wearable device 130, and the mobile terminal device 140 through the network.

For ease of presentation, the motion data calibration system 180 located in the processing device 110 is hereinafter used as an example in the following description.

In some embodiments, the motion monitoring system 100 may further include a database. The database may store data (for example, an initially set threshold condition) and/or an instruction (for example, a feedback instruction). In some embodiments, the database may store data obtained from the wearable device 130 and/or the mobile terminal device 140. In some embodiments, the database may store information and/or instructions for execution or use by the processing device 110, to perform the exemplary method described in this application. In some embodiments, the database may be connected to the network 120 to communicate with one or more components of the motion monitoring system 100 (for example, the processing device 110, the wearable device 130, and the mobile terminal device 140). Through the network 120, the one or more components of the motion monitoring system 100 may access data or instructions stored in the database. In some embodiments, the database may be directly connected to or communicate with the one or more components of the motion monitoring system 100. In some embodiments, the database may be a part of the processing device 110.

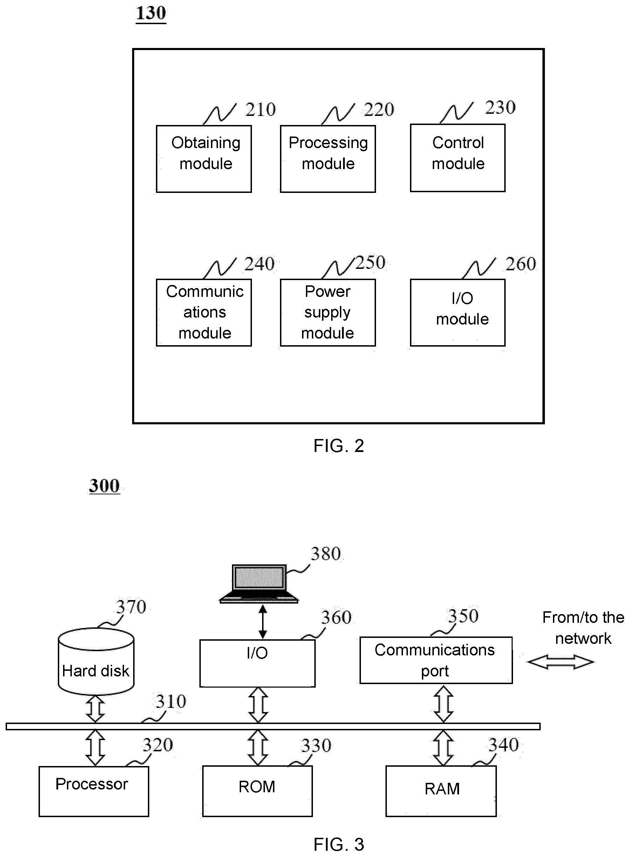

FIG. 2 is an exemplary schematic diagram of hardware and/or software of a wearable device 130 according to some embodiments of this application. As shown in FIG. 2, the wearable device 130 may include an obtaining module 210, a processing module 220 (also referred to as a processor), a control module 230 (also referred to as a main controller, an MCU, or a controller), a communications module 240, a power supply module 250, and an input/output module 260.

The obtaining module 210 may be configured to obtain an action signal during a movement of a user. In some embodiments, the obtaining module 210 may include a sensor unit, where the sensor unit may be configured to obtain one or more action signals during movement of the user. In some embodiments, the sensor unit may include, but is not limited to, one or more of a myoelectric sensor, a posture sensor, an electrocardio sensor, a respiration sensor, a temperature sensor, a humidity sensor, an inertial sensor, a blood oxygen saturation sensor, a Hall sensor, a galvanic skin sensor, a rotation sensor, and the like. In some embodiments, the action signal may include one or more of a myoelectric signal, a posture signal, an electrocardio signal, a respiratory frequency signal, a temperature signal, a humidity signal, and the like. The sensor unit may be placed in different positions of the wearable device 130 depending on a type of an action signal to be obtained. For example, in some embodiments, the myoelectric sensor (also referred to as an electrode element) may be disposed at a position of a muscle of a human body, and the myoelectric sensor may be configured to capture a myoelectric signal during movement of the user. The myoelectric signal and feature information (for example, frequency information or amplitude information) corresponding to the myoelectric signal may reflect a status of the muscle during movement of the user. The posture sensor may be disposed at different positions of the human body (for example, positions corresponding to a torso, four limbs, and joints, in the wearable device 130), and the posture sensor may be configured to capture a posture signal during movement of the user. The posture signal and feature information (for example, a direction of an angular velocity, an angular velocity value, an acceleration value of the angular velocity, an angle, displacement information, and stress) corresponding to the posture signal may reflect a posture during movement of the user. The electrocardio sensor may be disposed at a position around a chest of the human body, and the electrocardio sensor may be configured to capture electrocardio data during movement of the user. The respiration sensor may be disposed at a position around the chest of the human body, and the respiration sensor may be configured to capture respiratory data (for example, a respiratory frequency and a respiratory amplitude) during movement of the user. The temperature sensor may be configured to capture temperature data (for example, a shell temperature) during movement of the user. The humidity sensor may be configured to capture humidity data of an external environment during movement of the user.

The processing module 220 may process data from the obtaining module 210, the control module 230, the communications module 240, the power supply module 250, and/or the input/output module 260. For example, the processing module 220 may process the action signal during movement of the user from the obtaining module 210. In some embodiments, the processing module 220 may preprocess the action signal (for example, the myoelectric signal or the posture signal) obtained by the obtaining module 210. For example, the processing module 220 performs segmentation processing on the myoelectric signal or the posture signal during movement of the user. In another example, the processing module 220 may perform preprocessing (for example, filtering processing or signal correction processing) on the myoelectric signal during movement of the user, to improve quality of the myoelectric signal. In another example, the processing module 220 may determine, based on the posture signal during movement of the user, feature information corresponding to the posture signal. In some embodiments, the processing module 220 may process an instruction or an operation from the input/output module 260. In some embodiments, the processed data may be stored in a memory or a hard disk. In some embodiments, the processing module 220 may transmit, through the communications module 240 or a network 120, the data processed by the processing module 220 to one or more components of a motion monitoring system 100. For example, the processing module 220 may send a motion monitoring result of the user to the control module 230, and the control module 230 may execute a subsequent operation or instruction based on an action determining result.

The control module 230 may be connected to other modules in the wearable device 130. In some embodiments, the control module 230 may control a running status of another module in the wearable device 130. For example, the control module 230 may control a power supply status (for example, a normal mode or a power saving mode), a power supply time, and the like of the power supply module 250. In another example, the control module 230 may control the input/output module 260 based on the action determining result of the user, and may further control a mobile terminal device 140 to send a motion feedback result of the user to the user. When there is a problem with the action (for example, the action does not conform to a standard) during movement of the user, the control module 230 may control the input/output module 260, and may further control the mobile terminal device 140 to provide feedback for the user, so that the user can learn the movement status of the user and adjust the action. In some embodiments, the control module 230 may further control one or more sensors in the obtaining module 210 or another module to provide feedback about the human body. For example, when the force of a muscle is excessive during movement of the user, the control module 230 may control an electrode module at a position of the muscle to electrically stimulate the user to prompt the user to adjust the action in time.

In some embodiments, the communications module 240 may be configured to exchange information or data. In some embodiments, the communications module 240 may be used for communication between internal components of the wearable device 130. For example, the obtaining module 210 may send an action signal (for example, the myoelectric signal or the posture signal) of the user to the communications module 240, and the communications module 240 may send the action signal to the processing module 220. In some embodiments, the communications module 240 may be further used for communication between the wearable device 130 and other components of the motion monitoring system 100. For example, the communications module 240 may send status information (for example, a switch status) of the wearable device 130 to a processing device 110, and the processing device 110 may monitor the wearable device 130 based on the status information. The communications module 240 may use wired, wireless, or hybrid wired and wireless technologies.

In some embodiments, the power supply module 250 may supply power to other components of the motion monitoring system 100.

The input/output module 260 may obtain, transmit, and send signals. The input/output module 260 may be connected to or communicate with other components of the motion monitoring system 100. The other components of the motion monitoring system 100 may be connected or communicate through the input/output module 260.

It should be noted that the foregoing description of the motion monitoring system 100 and modules thereof is merely for ease of description and is not intended to limit one or more embodiments of this application to the scope of the illustrated embodiments. It may be understood that, after learning the principle of the system, a person skilled in the art may combine the modules, or connect a constituent subsystem to other modules, or omit one or more modules, without departing the principle. For example, the obtaining module 210 and the processing module 220 may be one module, and the module may have functions for obtaining and processing the action signal of the user. In another example, alternatively, the processing module 220 may not be disposed in the wearable device 130, but is integrated with the processing device 110. All such variations shall fall within the scope of protection of one or more embodiments of this application.

FIG. 3 is an exemplary schematic diagram of hardware and/or software of a computing device 300 according to some embodiments of this application. In some embodiments, a processing device 110 and/or a mobile terminal device 140 may be implemented on the computing device 300. In some embodiments, a motion data calibration system 180 may be implemented on the computing device 300. As shown in FIG. 3, the computing device 300 may include an internal communications bus 310, at least one processor 320, at least one storage medium, at least one communications port 350, an input/output interface 360, and a user interface 380.

The internal communications bus 310 may implement data communication between various components in the computing device 300. For example, the at least one processor 320 may send data to other hardware such as the at least one storage medium or the input/output port 360 through the internal communications bus 310. In some embodiments, the internal communications bus 310 may be an industry standard (ISA) bus, an extended industry standard (EISA) bus, a video electronics standard (VESA) bus, a peripheral component interconnect standard (PCI) bus, or the like. In some embodiments, the internal communications bus 310 may be used to connect various modules (for example, the obtaining module 210, the processing module 220, the control module 230, the communications module 240, and the input/output module 260) in the motion monitoring system 100 shown in FIG. 1.

The at least one storage medium of the computing device 300 may include a data storage apparatus. The data storage apparatus may be a non-transitory storage medium, or may be a transitory storage medium. For example, the data storage apparatus may include one or more of a read-only memory (ROM) 330, a random access memory (RAM) 340, a hard disk 370, and the like. The example ROM may include a mask ROM (MROM), a programmable ROM (PROM), an erasable programmable ROM (PEROM), an electronic erasable programmable ROM (EEPROM), an optical disc (CD-ROM), a digital versatile disk ROM, or the like. The example RAM may include a dynamic RAM (DRAM), a double date rate synchronous dynamic RAM (DDR SDRAM), a static RAM (SRAM), a thyristor RAM (T-RAM), a zero-capacitance RAM (Z-RAM), or the like. The storage medium may store data/information obtained from any other component of the motion monitoring system 100. The storage medium further includes at least one instruction set stored in the data storage apparatus. The instruction is computer program code, and the computer program code may include a program, a routine, an object, a component, a data structure, a process, a module, and the like that perform the motion data calibration method provided in this specification. In some embodiments, the storage medium of the computing device 300 may be located in the wearable device 130, or may be located in the processing device 110.

The at least one processor 320 may be communicatively connected to the at least one storage medium. The at least one processor 320 is configured to execute the at least one instruction set. When the computing device 300 runs, the at least one processor 320 may read the at least one instruction set, and execute a computer instruction (program code) based on an instruction of the at least one instruction set, so as to perform a function of the motion monitoring system 100 described in this application. The processor 320 may perform all steps included in the data processing method. The computer instruction may include a program, an object, a component, a data structure, a process, a module, and a function (the function is a particular function described in this application). For example, the processor 320 may process an action signal (for example, a myoelectric signal or a posture signal) obtained from a wearable device 130 or/and a mobile terminal device 140 of the motion monitoring system 100 during a movement of a user, and monitor a movement action of the user based on the action signal during movement of the user. For example, the processor 320 may be configured to process the action data during movement of the user obtained from the wearable device 130 or/and the mobile terminal device 140 of the motion monitoring system 100, and perform the motion data calibration method described in this specification based on the instruction of the at least one instruction set to convert the action data to two-dimensional posture data. In some embodiments, the processor 320 may include a microcontroller, a microprocessor, a reduced instruction set computer (RISC), an application-specific integrated circuit (ASIC), an application-specific instruction set processor (ASIP), a central processing unit (CPU), a graphics processing unit (GPU), a physical processing unit (PPU), a microcontroller unit, a digital signal processor (DSP), a field programmable gate array (FPGA), an advanced reduced instruction set computing machine (ARM), a programmable logic device, any circuit or processor that can implement one or more functions, and the like, or any combination thereof. For a purpose of description only, only one processor 320 is depicted in the computing device 300 in FIG. 3. However, it should be noted that the computing device 300 in this application may also include a plurality of processors 320.

The hard disk 370 may be configured to store information and data generated by the processing device 110 or received from the processing device 110. For example, the hard disk 370 may store user confirmation information of the user. In some embodiments, the hard disk 370 may be disposed in the processing device 110 or the wearable device 130.

The user interface 380 may implement interaction and information exchange between the computing device 300 and a user. In some embodiments, the user interface 380 may be configured to present a motion record generated by the motion monitoring system 100 to the user. In some embodiments, the user interface 380 may include a physical display, such as a display with a speaker, an LCD display, an LED display, an OLED display, or an electronic ink (E-Ink) display.

The input/output interface 360 may be configured to input or output signals, data, or information. In some embodiments, the input/output interface 360 may enable the user to interact with the motion monitoring system 100.

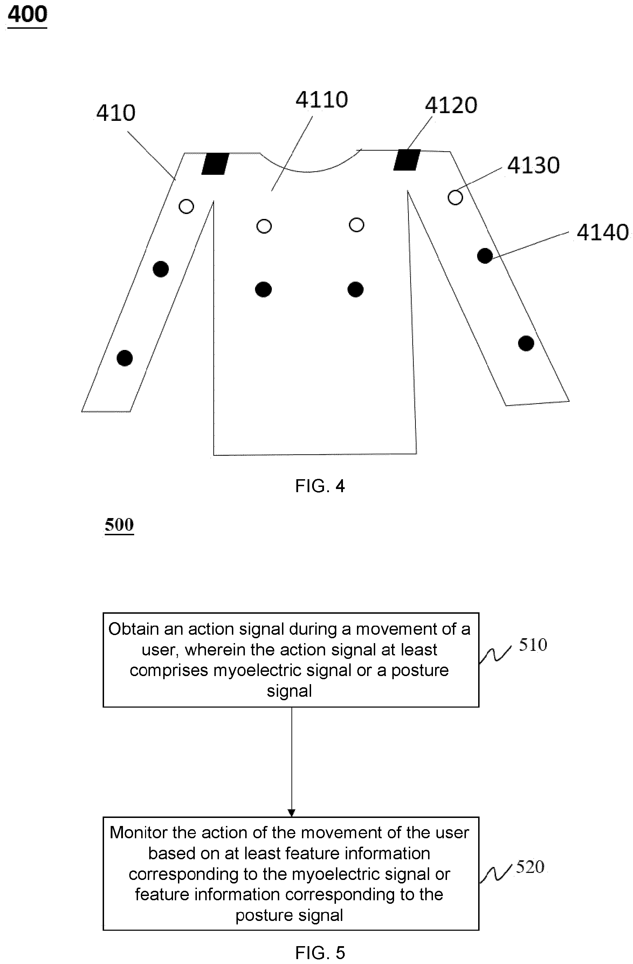

FIG. 4 is an exemplary structural diagram of a wearable device according to some embodiments of this application. To further describe the wearable device, an upper garment is used for illustration. As shown in FIG. 4, a wearable device 400 may include an upper garment 410. The upper garment 410 may include an upper garment base 4110, at least one upper garment processing module 4120, at least one upper garment feedback module 4130, at least one upper garment obtaining module 4140, and the like. The upper garment base 4110 may be a garment worn on an upper part of a human body. In some embodiments, the upper garment base 4110 may include a short-sleeved T-shirt, a long-sleeve T-shirt, a shirt, a coat, or the like. The at least one upper garment processing module 4120 and the at least one upper garment obtaining module 4140 may be located in areas of the upper garment base 4110 that fit onto different parts of the human body. The at least one upper garment feedback module 4130 may be located in any position of the upper garment base 4110. The at least one upper garment feedback module 4130 may be configured to provide feedback of movement status information of an upper part of the body of a user. Exemplary feedback manners may include, but are not limited to, a voice prompt, a text prompt, a pressure prompt, a current stimulation, and the like. In some embodiments, the at least one upper garment obtaining module 4140 may include, but is not limited to, one or more of a posture sensor, an electrocardio sensor, a myoelectric sensor, a temperature sensor, a humidity sensor, an inertial sensor, an acid-base sensor, an acoustic transducer, and the like. The sensor in the upper garment obtaining module 4140 may be placed in different positions of the body of the user based on different signals to be measured. For example, when the posture sensor is configured to obtain a posture signal during movement of the user, the posture sensor may be placed at positions corresponding to a torso, two arms, and joints of the human body, in the upper garment base 4110. In another example, when the myoelectric sensor is configured to obtain a myoelectric signal during movement of the user, the myoelectric sensor may be located near the user's muscle to be measured. In some embodiments, the posture sensor may include, but is not limited to, an acceleration three-axis sensor, an angular velocity three-axis sensor, a magnetic sensor, or the like, or any combination thereof. For example, one posture sensor may include an acceleration three-axis sensor or an angular velocity three-axis sensor. In some embodiments, the posture sensor may further include a strain sensor. The strain sensor may be a sensor that generates strain based on a forced deformation of an object to be measured. In some embodiments, the strain sensor may include, but is not limited to, one or more of a strain force sensor, a strain pressure sensor, a strain torque sensor, a strain displacement sensor, a strain acceleration sensor, and the like. For example, the strain sensor may be disposed at a joint position of the user, and a bending angle and a bending direction at the user's joint may be obtained by measuring a magnitude of resistance that varies with a tensile length in the strain sensor. It should be noted that the upper garment 410 may further include other modules, for example, a power supply module, a communications module, and an input/output module, in addition to the upper garment base 4110, the upper garment processing module 4120, the upper garment feedback module 4130, and the upper garment obtaining module 4140 described above. The upper garment processing module 4120 is similar to the processing module 220 in FIG. 2. The upper garment obtaining module 4140 is similar to the obtaining module 210 in FIG. 2. For detailed descriptions about the modules in the upper garment 410, reference may be made to related descriptions in FIG. 2 of this application. Details are not described herein again.

FIG. 5 is an exemplary flowchart of a motion monitoring method according to some embodiments of this application. As shown in FIG. 5, the process 500 may include the following steps.

Step 510: Obtain an action signal during a movement of a user.

In some embodiments, step 510 may be performed by the obtaining module 210. The action signal refers to human parameter information during movement of the user. In some embodiments, the human parameter information may include, but is not limited to, one or more of a myoelectric signal, a posture signal, an electrocardio signal, a temperature signal, a humidity signal, a blood oxygen concentration, a respiratory frequency, and the like. In some embodiments, a myoelectric sensor in the obtaining module 210 may capture a myoelectric signal during movement of the user. For example, when the user performs seated chest fly, a myoelectric sensor corresponding to a position of a pectoral muscle, a latissimus dorsi, or the like of a human body, in a wearable device may capture a myoelectric signal at a corresponding muscle position of the user. In another example, when the user performs a deep squat action, a myoelectric sensor corresponding to a position of a gluteus maximus, a quadriceps femoris, or the like of the human body, in the wearable device may capture a myoelectric signal at a corresponding muscle position of the user. In another example, when the user takes running exercise, a myoelectric sensor corresponding to a position of a gastrocnemius or the like of the human body, in the wearable device may capture a myoelectric signal at the position of the gastrocnemius or the like of the human body. In some embodiments, a posture sensor in the obtaining module 210 may capture a posture signal during movement of the user. For example, when the user performs barbell horizontal-pushing motion, a posture sensor corresponding to a position of a triceps brachii or the like of the human body, in the wearable device may capture a posture signal at the position of the triceps brachii or the like of the user. In another example, when the user performs a dumbbell bird action, a posture sensor diposed at a position of a deltoid or the like of the human body may capture a posture signal at the position of the deltoid or the like of the user. In some embodiments, the obtaining module 210 may include a plurality of posture sensors, and the plurality of posture sensors may obtain posture signals of a plurality of parts of the body during movement of the user, where the plurality of posture signals may reflect a status of relative movement between different parts of the human body. For example, a posture signal at an arm and a posture signal at a torso may reflect a movement status of the arm relative to the torso. In some embodiments, posture signals are associated with types of posture sensors. For example, when a posture sensor is an angular velocity three-axis sensor, an obtained posture signal is angular velocity information. In another example, when posture sensors are an angular velocity three-axis sensor and an acceleration three-axis sensor, obtained posture signals are angular velocity information and acceleration information. In another example, when a posture sensor is a strain sensor, the strain sensor may be disposed at a joint position of the user, a posture signal obtained by measuring a magnitude of resistance varying with a tensile length in the strain sensor may be displacement information, stress, and the like, and the posture signal may represent a bending angle and a bending direction at the user's joint. It should be noted that parameter information that can be used to reflect relative motion of the body of the user may all be feature information corresponding to posture signals, and different types of posture sensors may be used to obtain the information based on feature information types.

In some embodiments, the action signal may include a myoelectric signal of a specific part of the body of the user and a posture signal of the specific part. The myoelectric signal and the posture signal may reflect a movement status of the specific part of the body of the user from different perspectives. In short, the posture signal of the specific part of the body of the user may reflect an action type, an action amplitude, an action frequency, or the like of the specific part. The myoelectric signal may reflect a muscle status of the specific part during movement. In some embodiments, myoelectric signals and/or posture signals of the same part of the body may be used to better evaluate whether the action of the part meets the standard.

Step 520: Monitor the action of the movement of the user based on at least feature information corresponding to the myoelectric signal or feature information corresponding to the posture signal.

In some embodiments, this step may be performed by the processing module 220 and/or the processing device 110. In some embodiments, the feature information corresponding to the myoelectric signal may include, but is not limited to, one or more of frequency information, amplitude information, and the like. The feature information corresponding to the posture signal is parameter information used to represent relative motion of the body of the user. In some embodiments, the feature information corresponding to the posture signal may include, but is not limited to, one or more of a direction of an angular velocity, an angular velocity value, an acceleration value of the angular velocity, and the like. In some embodiments, the feature information corresponding to the posture signal may further include an angle, displacement information (for example, a tensile length in a strain sensor), stress, and the like. For example, when a posture sensor is a strain sensor, the strain sensor may be disposed at a joint position of the user, a posture signal obtained by measuring a magnitude of resistance varying with a tensile length in the strain sensor may be displacement information, stress, and the like, and the posture signal may represent a bending angle and a bending direction at the user's joint. In some embodiments, the processing module 220 and/or the processing device 110 may extract the feature information (for example, the frequency information and the amplitude information) corresponding to the myoelectric signal or the feature information (for example, the direction of the angular velocity, the angular velocity value, the acceleration value of the angular velocity, the angle, the displacement information, stress, and the like) corresponding to the posture signal, and monitor the action of the user based on the feature information corresponding to the myoelectric signal or the feature information corresponding to the posture signal. Herein, the monitoring of the action of the movement of the user includes monitoring information related to the action of the user. In some embodiments, the information related to the action may include one or more of an action type of the user, a quantity of actions, action quality (for example, whether the action of the user conforms to a standard), an action time, and the like. The action type refers to an exercise action taken during movement of the user. In some embodiments, the action type may include, but is not limited to, one or more of seated chest fly, deep squat exercise, hard pull exercise, plank exercise, running, swimming, and the like. The action quantity is a quantity of actions performed during movement of the user. For example, the user performs seated chest fly for 10 times, and the action quantity herein is 10. The action quality refers to a degree of conformity of an exercise action performed by the user to a standard exercise action. For example, when the user performs a deep squat action, the processing device 110 may determine an action type of the action of the user based on feature information corresponding to an action signal (myoelectric signal and posture signal) at a specific muscle position (gluteus maximus, quadriceps femoris, or the like), and determine action quality of the deep squat action of the user based on an action signal of a standard deep squat action. The action time refers to the time corresponding to one or more action types of the user or the total time of the motion process.

As described above, the feature information corresponding to the posture signal may include parameter information that can be used to reflect relative motion of the body of the user. To monitor the action of the movement of the user, the motion monitoring system 100 needs to obtain relative motion between different parts of the body of the user. As above, the posture signal may be posture data obtained by a posture sensor. The posture sensors may be distributed at different parts of the body of the user. To obtain relative motion between a plurality of different parts of the body of the user, the motion monitoring system 100 may calibrate posture signals between the plurality of different parts of the body of the user.

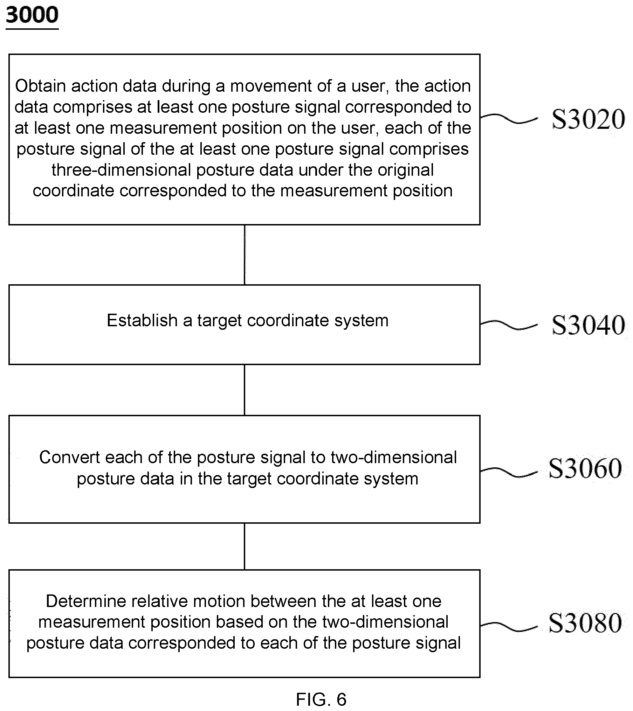

FIG. 6 is an exemplary flowchart of a motion data calibration method 3000 according to some embodiments of this application. As above, the motion data calibration system 180 may perform the motion data calibration method 3000 provided in this application. Specifically, the motion data calibration system 180 may perform the motion data calibration method 3000 on the processing device 110, or may perform the motion data calibration method 3000 on the mobile terminal device 140. For ease of presentation, an example in which the motion data calibration system 180 performs the motion data calibration method 3000 on the processing device 110 is used for description. Specifically, the processor 320 in the processing device 110 may read an instruction set stored in a local storage medium thereof, and then perform the motion data calibration method 3000 provided in this application. The motion data calibration method 3000 may include the following steps.

S3020. Obtain action data during a movement of a user.

In some embodiments, this step may be performed by the processing module 220 and/or the processing device 110. The action data refers to human motion parameter information during movement of the user. In some embodiments, the action data may include at least one posture signal corresponding to at least one measurement position on the body of the user. The posture signal and feature information (for example, a direction of an angular velocity, an angular velocity value, an acceleration value of the angular velocity, an angle, displacement information, and stress) corresponding to the posture signal may reflect a posture during movement of the user. The at least one measurement position corresponds to the at least one posture signal on a one-to-one basis. Measurement positions may be different parts of the body of the user. The at least one posture signal corresponds to an actual posture of the at least one measurement position on the body of the user during movement of the user. Each of the posture signal of the at least one posture signal may include three-dimensional posture data of the measurement position corresponding to the posture signal in an original coordinate system. The original coordinate system may be a coordinate system in which the posture signal is located. When the posture of the user does not change, a change of the original coordinate system may also cause a change of the posture signal. Each of the posture signal may include one or more types of three-dimensional posture data, for example, three-dimensional angle data, three-dimensional angular velocity data, three-dimensional angular acceleration data, three-dimensional velocity data, three-dimensional displacement data, and three-dimensional stress data.

In some embodiments, a posture signal may be obtained by a posture sensor in the wearable device 130. As described above, the sensor unit of the obtaining module 210 in the wearable device 130 may include a posture sensor. Specifically, the wearable device 130 may include at least one posture sensor. The at least one posture sensor may be located in at least one measurement position on the body of the user. The posture sensor may capture a posture signal of the corresponding measurement position on the body of the user. Posture sensors in the wearable device 130 may be distributed on four limbs of the human body (for example, arms and legs), the torso of the human body (for example, the chest, abdomen, back, and waist), the head of the human body, and the like. The posture sensors can capture posture signals at other parts of the human body, such as the limbs and the torso. The posture sensors may be placed at different positions of the wearable device 130 based on the posture signals to be obtained, to measure the posture signals corresponding to different positions of the human body. In some embodiments, a posture sensor may also be a sensor having an attitude and heading reference system (AHRS) with a posture fusion algorithm. The posture fusion algorithm may fuse data of a nine-axis inertial measurement unit (IMU) having a three-axis acceleration sensor, three-axis angular velocity sensor, and a three-axis geomagnetic sensor into an Euler angle or quaternion, to obtain a posture signal of a body part of the user in which the posture sensor is located. In some embodiments, the processing module 220 and/or the processing device 110 may determine, based on the posture signal, the feature information corresponding to the posture signal. In some embodiments, the feature information corresponding to the posture signal may include, but is not limited to, an angular velocity value, a direction of an angular velocity, an acceleration value of the angular velocity, and the like. In some embodiments, the posture sensor may be a strain sensor, and the strain sensor may obtain a bending direction and a bending angle at the user's joint to obtain a posture signal during movement of the user. For example, the strain sensor may be disposed at a knee joint of the user, during movement of the user, a body part of the user acts on the strain sensor, and a bending direction and a bending angle at the knee joint of the user may be calculated based on a resistance or length change of the strain sensor, to obtain a posture signal of a leg of the user. In some embodiments, the posture sensor may further include a fiber sensor, and the posture signal may be represented by a change of a direction of light after bending at the fiber sensor. In some embodiments, the posture sensor may alternatively be a magnetic flux sensor, and the posture signal may be represented by a change of a magnetic flux. It should be noted that the type of the posture sensor is not limited to the foregoing sensors, but may also be other sensors. All sensors capable of obtaining a posture signal of a user shall fall within the scope of the posture sensor in this application.

As described above, each of the posture signal may include one or more types of three-dimensional posture data. The posture sensors may also include a variety of sensors. In some embodiments, the posture sensor may include at least one of an acceleration sensor, an angular velocity sensor, and a magnetic sensor.

When the posture signal is data measured by the posture sensor, the original coordinate system may be a coordinate system in which the posture sensor is located. In some embodiments, the original coordinate system is a coordinate system corresponding to a posture sensor disposed on the human body. When the user uses the wearable device 130, the posture sensors in the wearable device 130 are distributed at different parts of the human body, so that installation angles of the posture sensors on the human body are different, and the posture sensors at different parts respectively use coordinate systems of their own device bodies as original coordinate systems. Therefore, the posture sensors at different parts have different original coordinate systems. In some embodiments, a posture signal obtained by each posture sensor may be an expression under an original coordinate system of the corresponded posture sensor. The posture signal obtained by the posture sensor may be a posture signal of a preset fixed coordinate system in the original coordinate system. The preset fixed coordinate system may be a geodetic coordinate system, or may be any other preset coordinate system. A conversion relationship between the original coordinate system and the preset fixed coordinate system may be pre-stored in the motion data calibration system 180.

In some embodiments, the posture signal may be a signal directly obtained by the posture sensor, or may be a posture signal formed after a signal processing procedure such as conventional filtering, rectification, and wavelet transformation, or burr processing is performed on the signal directly obtained by the posture sensor, or may be a signal obtained by permutating or combining any one or more of the foregoing processing procedures.

In some embodiments, the posture signal may be data obtained by an image sensor. The image sensor may be an image sensor capable of obtaining depth information, such as a 3D structured light camera or a binocular camera. The image sensor may be installed at any position capable of shooting an image during movement of the user. There may be one or more image sensors. When there is a plurality of image sensors, the plurality of image sensors may be installed at a plurality of different positions. The image sensor may obtain a depth image during movement of the user. The depth image may include depth information of at least one measurement position on the body of the user relative to a coordinate system in which the image sensor is located. When the user moves, the motion data calibration system 180 can obtain a posture signal at each of the at least one measurement position by calculation based on a change in a plurality of frames of depth images. As described above, each of the posture signal may include one or more types of three-dimensional posture data. The motion data calibration system 180 may obtain different three-dimensional posture data through calculation.

When the posture signal is data measured by the image sensor, the original coordinate system may be a coordinate system in which the image sensor itself is located. In some embodiments, the posture signal obtained by the image sensor may be an expression in a coordinate system (original coordinate system) of the image sensor corresponding to the posture signal. The image sensor may perform calibration in advance. To be specific, a conversion relationship between the coordinate system of the image sensor and the foregoing preset fixed coordinate system may be pre-stored in the motion data calibration system 180.

For ease of presentation, in the following description, an example in which the action data is data measured by at least one posture sensor is used for description. For ease of presentation, the original coordinate system is defined as an o-xyz coordinate system, where o is a coordinate origin of the original coordinate system o-xyz, and an x-axis, a y-axis and a z-axis are respectively three mutually perpendicular coordinate of the original coordinate system o-xyz.

As described above, each posture signal may be three-dimensional posture data of the measurement position corresponding to the posture signal in the original coordinate system o-xyz. In some embodiments, the three-dimensional posture data may be posture data on three mutually perpendicular coordinate axes in the coordinate system in which the three-dimensional posture data is located. In some embodiments, the posture data may include angle data and angular velocity data. In some embodiments, the three-dimensional posture data in the original coordinate system o-xyz may include angle data and angular velocity data on the three mutually perpendicular coordinate axes: the x-axis, the y-axis, and the z-axis. For ease of presentation, the three-dimensional posture data of each posture signal in the original coordinate system o-xyz is respectively marked as three-dimensional angle (Euler) data $E_{sens}$ and three-dimensional angular velocity (Gyro) data $G_{sens}$. The three-dimensional angle data $E_{sens}$ may include angle data $E_{sens\_x}$ on the x-axis, angle data $E_{sens\_y}$ on the y-axis, and angle data $E_{sens\_z}$ on the z-axis. The three-dimensional angular velocity data $G_{sens}$ may include angular velocity data $G_{sens\_x}$ on the x-axis, angular velocity data $G_{sens\_y}$ on the y-axis, and angular velocity data $G_{sens\_z}$ on the z-axis.

S3040. Establish a target coordinate system.

In some embodiments, this step may be performed by the processing module 220 and/or the processing device 110. For ease of presentation, the target coordinate system is defined as O-XYZ. To conveniently determine relative motion between different parts of the user, the motion data calibration system 180 may convert the action data to posture data in a same known coordinate system (for example, the target coordinate system is defined as O-XYZ). The target coordinate system O-XYZ may include three mutually perpendicular coordinate axes: an X-axis, a Y-axis, and a Z-axis.

In some embodiments, the target coordinate system O-XYZ may be any calibrated coordinate system. In some embodiments, the target coordinate system O-XYZ may be the foregoing preset fixed coordinate system. In some embodiments, the target coordinate system O-XYZ and the foregoing preset fixed coordinate system may be different coordinate systems. A conversion relationship between the foregoing preset fixed coordinate system and the target coordinate system may be pre-stored in the motion data calibration system 180.

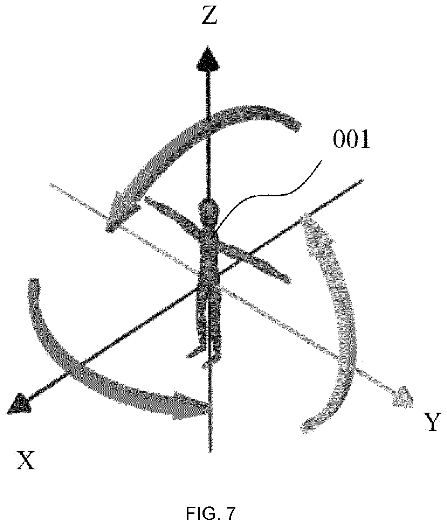

The motion data calibration system 180 is configured to calibrate the action data during movement of the user, and a measurement object thereof is the user. Therefore, in some embodiments, a length direction of the torso when the human body is standing may be used as the Z-axis in the target coordinate system O-XYZ. To be specific, the Z-axis is a direction opposite to a vertical direction of gravity acceleration. In other words, the Z-axis is a coordinate axis perpendicular to the ground and pointing to the sky. A plane formed by the X-axis and the Y-axis is a horizontal plane perpendicular to the Z-axis. In some embodiments, the X-axis and the Y-axis may be any two mutually perpendicular coordinate axes in the horizontal plane perpendicular to the Z-axis. In some embodiments, the X-axis may be an east-west coordinate axis, such as an east coordinate axis, and the Y-axis may be a north-south coordinate axis, such as a north coordinate axis. FIG. 7 is a schematic diagram of the target coordinate system O-XYZ according to an embodiment of this specification. A direction of the X-axis is just the front of a user 001 when the user is standing.

S3060. Convert each posture signal to two-dimensional posture data in the target coordinate system.

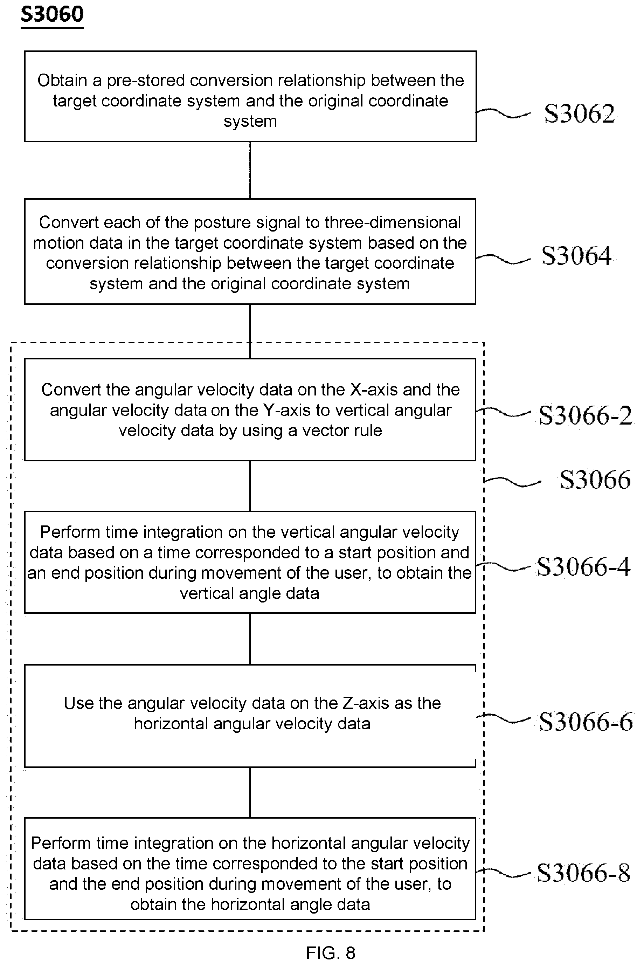

FIG. 8 is an exemplary flowchart for converting to two-dimensional posture data according to some embodiments of this application. FIG. 8 corresponds to step S3060. As shown in FIG. 8, step S3060 may include:

S3062. Obtain a pre-stored conversion relationship between the target coordinate system O-XYZ and the original coordinate system o-xyz.

As described above, the posture signal obtained by each posture sensor may be an expression in the original coordinate system corresponding to the posture sensor. Specifically, the posture signal obtained by the posture sensor may be a posture signal of the preset fixed coordinate system in the original coordinate system at the measurement position corresponding to the current posture signal. The motion data calibration system 180 may obtain, in a reverse conversion manner based on each posture signal, an expression of the original coordinate system o-xyz of the posture sensor corresponding to the posture signal in the preset fixed coordinate system. As described above, the conversion relationship between the preset fixed coordinate system and the target coordinate system O-XYZ may be pre-stored in the motion data calibration system 180. The motion data calibration system 180 may calculate and determine the conversion relationship between each original coordinate system o-xyz and the target coordinate system O-XYZ based on the conversion relationship between the preset fixed coordinate system and the target coordinate system O-XYZ. The motion data calibration system 180 may convert posture information in the original coordinate system o-xyz to posture information in the target coordinate system O-XYZ based on the conversion relationship. In some embodiments, the conversion relationship may be represented by one or more rotation matrices. The rotation matrix may be pre-stored in the motion data calibration system 180.

S3064. Convert each posture signal to three-dimensional motion data in the target coordinate system O-XYZ based on the conversion relationship between the target coordinate system O-XYZ and the original coordinate system o-xyz.

As described above, each posture signal may include three-dimensional posture data $E_{sens\_x}$, $E_{sens\_y}$, $E_{sens\_z}$, $G_{sens\_x}$, $G_{sens\_y}$, and $G_{sens\_z}$ in the original coordinate system o-xyz corresponding to the posture signal. The motion data calibration system 180 may determine, based on the conversion relationship between the target coordinate system O-XYZ and the original coordinate system o-xyz, three-dimensional motion data of the original coordinate system o-xyz at the measurement position corresponding to each posture signal in the target coordinate system O-XYZ. In some embodiments, the three-dimensional motion data includes at least angular velocity data $G_{global\_X}$ on the X-axis, angular velocity data $G_{global\_Y}$ on the Y-axis, and angular velocity data $G_{global\_Z}$ on the Z-axis.

S3066. Convert the three-dimensional motion data in the target coordinate system O-XYZ to two-dimensional posture data in the target coordinate system O-XYZ.

The two-dimensional posture data is data in a two-dimensional coordinate system. The two-dimensional coordinate system in the target coordinate system O-XYZ may include a motion plane when limbs of the user 001 swing and a motion plane when the torso of the user 001 rotates. In some embodiments, the two-dimensional posture data in the target coordinate system O-XYZ may include horizontal posture data and vertical posture data. The horizontal posture data may include horizontal angle data $E_{global\_Z}$ and horizontal angular velocity data $G_{global\_Z}$ during a movement in a horizontal plane perpendicular to the Z-axis. The vertical posture data may include vertical angle data $E_{global\_XY}$ and vertical angular velocity data $G_{global\_XY}$ during a movement in any vertical plane perpendicular to the horizontal plane. The vertical plane may be any plane perpendicular to the horizontal plane. The horizontal angle data $E_{global\_Z}$ may be a rotational angle of the measurement position in the horizontal plane in the target coordinate system O-XYZ. The horizontal angular velocity data $G_{global\_Z}$ may be a rotational angular velocity of the measurement position in the horizontal plane in the target coordinate system O-XYZ. The vertical angle data $E_{global\_XY}$ may be a rotational angle of the measurement position in any vertical plane in the target coordinate system O-XYZ. The vertical angular velocity data $G_{global\_XY}$ may be a rotational angular velocity of the measurement position in any vertical plane in the target coordinate system O-XYZ.

Because for most exercise actions in a gymnasium, a major portion of an action of the user 001 may be decomposed to motion in two planes, for example, motion in place in the horizontal plane and motion in any one vertical plane. Different actions performed by the user 001 during movement of the user 001 can be distinguished only by the motion on the horizontal plane and the motion on the vertical plane. For example, while the user 001 is running on a treadmill, a running action of the user 001 is mainly focused on rotational movement around joints parallel to the horizontal plane. In this case, the rotational movement occurs in a vertical plane perpendicular to the horizontal plane, and the vertical plane extends in a direction of a body orientation of the user 001. A center of gravity of the user 001 moves linearly up and down in this vertical plane, and each limb of the user swings with the center of gravity. For example, when the user 001 performs an action of biceps lifting, the action of biceps lifting includes only movement in the vertical plane. In another example, when the user 001 performs an action of seated chest fly, the action of seated chest fly includes only an action in the horizontal plane.

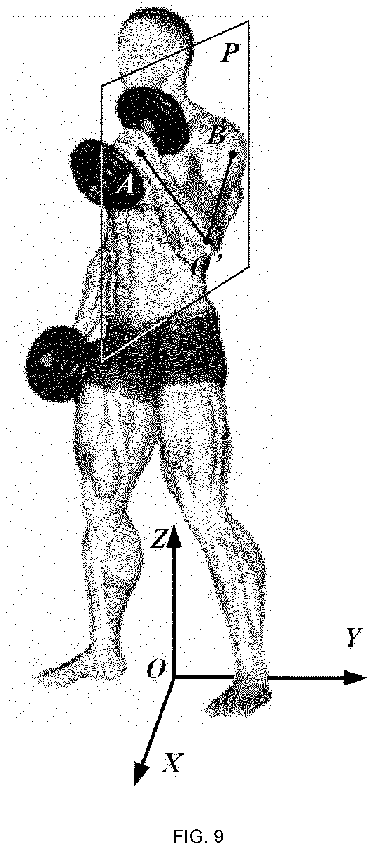

FIG. 9 is a diagram of a coordinate system during movement of the user 001 according to some embodiments of this application. In FIG. 9, when the user 001 performs biceps lifting, the orientation of the user 001 is basically unchanged. The action of biceps lifting is mainly focused on a vertical plane P formed by a forearm AO' and an upper arm O'B. The plane P is generally perpendicular to the horizontal plane X-Y due to an action of overcoming gravity of a dumbbell. In the vertical plane P, a pose of the upper arm O'B is basically unchanged, and the forearm AO' swings back and forth around the elbow O'. A vector direction of the swing is a normal direction of the vertical plane P. In addition, the action of biceps lifting of the user 001 has negligible components in other directions. Based on the foregoing analysis, subsequently, the motion data calibration system 180 converts the three-dimensional motion data to two-dimensional posture data.

As shown in FIG. 8, step S3066 may include:

S3066-2. Convert the angular velocity data $G_{global\_X}$ on the X-axis and the angular velocity data $G_{global\_Y}$ on the Y-axis to vertical angular velocity data $G_{global\_XY}$ by using a vector rule.

The vertical angular velocity data $G_{global\_XY}$ may be expressed as the following formula:

$$|G_{globaal\_XY}| = \sqrt{G_{global\_X}{}^2 + G_{global\_Y}{}^2} \quad \text{formula (1)}$$

$$G_{globaal\_XY} = |G_{globaal\_XY}| * a \quad \text{formula (2)}$$

$$a = \begin{cases} 1, & \text{where a larger one of absolute values of } G_{sens\_x} \text{ and } G^{sens\_y} \text{ is greater than 0} \\ -1, & \text{where a larger one of absolute values of } G^{sens\_x} \text{ and } G^{sens\_y} \text{ is less than 0} \end{cases}$$
formula (3)

S3066-4. Perform time integration on the vertical angular velocity data based on a time corresponding to a start position and a time corresponding to an end position during movement of the user 001, to obtain the vertical angle data $E_{global\_XY}$.

The vertical angle data $E_{global\_XY}$ may be expressed as the following formula:

$$E_{global\_XY} = \int_{startpos}^{endpos} G_{globaal\_XY} \cdot dt \qquad \text{formula (4)}$$

where startpos and endpos are a start time and an end time corresponding to a start position (start position) and an end position (end position) of an action.

S3066-6. Use the angular velocity data $G_{global\_Z}$ on the Z-axis as the horizontal angular velocity data $G_{global\_Z}$.

S3066-8. Perform time integration on the horizontal angular velocity data $G_{global\_Z}$ based on the time corresponding to the start position and the time corresponding to the end position during movement of the user 001, to obtain the horizontal angle data $E_{global\_Z}$.

The horizontal angle data $E_{global\_Z}$ may be expressed as the following formula:

$$E_{global\_Z} = \int_{startpos}^{endpos} G_{globaal\_Z} \cdot dt \qquad \text{formula (5)}$$

where startpos and endpos are a start time and an end time corresponding to a start position (start position) and an end position (end position) of an action.

In some embodiments, the motion data calibration method 3000 may further include:

S3080. Determine relative motion between the at least one measurement position based on the two-dimensional posture data corresponded to each posture signal.

The motion data calibration system 180 may determine relative motion between different movement parts of the body of the user 001 by using at least one piece of two-dimensional posture data corresponding to at least one posture signal corresponding to the at least one measurement position on the body of the user 001. For example, the relative motion between the arms and the torso during movement of the user 001 may be determined by using feature information corresponding to posture sensors at the arms of the user 001 and feature information corresponding to a posture sensor at the torso of the user 001.

In summary, the motion data calibration method 3000 and the system 180 provided in this application can convert the action data during movement of the user 001 from the three-dimensional posture data on the three mutually perpendicular coordinate axes to the two-dimensional data in the target coordinate system, that is, the posture data in the horizontal plane and the posture data in the vertical plane, thereby dividing the action of the movement of the user 001 to the motion in the horizontal direction and the motion in the vertical direction, and avoiding a data discrepancy caused by different orientations of the user 001. The method 3000 and the system 180 may eliminate affection of the orientations of the user 001 on the motion data. Therefore, the method 3000 and the system 180 can calibrate the motion data without requiring the user 001 to perform a calibration action.

Basic concepts have been described above. Apparently, for a person skilled in the art, the foregoing detailed disclosure is only an example, and should not be construed as a limitation on this application. Although not explicitly described herein, various changes, improvements, and modifications to this application may be applied by a person skilled in the art. Such changes, improvements, and modifications are suggested in this application. Therefore, such changes, improvements, and modifications still fall within the spirit and scope of the exemplary embodiments of this application.

In addition, specific terms used in this application are to describe the embodiments of this application. For example, "one embodiment", "an embodiment", and/or "some embodiments" shall mean a feature, a structure, or a characteristic in connection with at least one embodiment of this application. Therefore, it should be emphasized and noted that two or more references to "an embodiment" or "one embodiment" or "an alternative embodiment" in various places throughout this application may not necessarily refer to the same embodiment. Furthermore, some of the features, structures, or characteristics in one or more embodiments of this application may be appropriately combined.

**Claims**

1. A method for calibrating motion data, **characterized in that** the method comprises:

obtaining action data during a movement of a user, wherein the action data includes at least one posture signal corresponding to at least one measurement position on a body of the user, and each posture signal of the at least one posture signal includes three-dimensional posture data of a corresponded measurement position in an original coordinate system;

establishing a target coordinate system, wherein the target coordinate system includes an X-axis, a Y-axis, and a Z-axis mutually perpendicular to each other; and

converting each posture signal to two-dimensional posture data in the target coordinate system.

2. The method according to claim 1, **characterized in that** each posture signal includes data obtained by a posture sensor, and the original coordinate system includes a coordinate system in which the posture sensor is located.

3. The method according to claim 2, **characterized in that** the posture sensor includes at least one of an acceleration sensor, an angular velocity sensor, or a magnetic sensor.

4. The method according to claim 1, **characterized in that** each posture signal includes data obtained by an image sensor, and the original coordinate system includes a coordinate system in which the image sensor is located.

5. The method according to claim 1, **characterized in that** the three-dimensional posture data includes angle data and angular velocity data on the X-axis, the Y-axis and the Z-axis mutually perpendicular to each other.

6. The method according to claim 1, **characterized in that** the converting of each posture signal to the two-dimensional posture data in the target coordinate system includes:

obtaining a pre-stored conversion relationship between the target coordinate system and the original coordinate system;

converting each posture signal to the three-dimensional motion data in the target coordinate system based on the conversion relationship, wherein the three-dimensional motion data includes at least angular velocity data on the X-axis, angular velocity data on the Y-axis, and angular velocity data on the Z-axis; and

converting the three-dimensional motion data in the target coordinate system to the two-dimensional posture data in the target coordinate system.

7. The method according to claim 6, **characterized in that** the Z-axis of the target coordinate system is in a direction opposite to a vertical direction of a gravity acceleration.

8. The method according to claim 7, **characterized in that** the two-dimensional posture data in the target coordinate system includes:

horizontal posture data, including horizontal angle data and horizontal angular velocity data during a movement of the user in a horizontal plane perpendicular to the Z-axis; and

vertical posture data, including vertical angle data and vertical angular velocity data during a movement of the user in any vertical plane perpendicular to the horizontal plane.

9. The method according to claim 8, **characterized in that** the converting of the three-dimensional motion data in the target coordinate system to the two-dimensional posture data in the target coordinate system includes:

converting the angular velocity data on the X-axis and the angular velocity data on the Y-axis to vertical angular velocity data by using a vector law;

performing time integration with the vertical angular velocity data based on a time corresponding to a start position and a time corresponding to an end position during the movement of the user, to obtain the vertical angle data;

using the angular velocity data on the Z-axis as the horizontal angular velocity data; and

performing time integration on the horizontal angular velocity data based on the time corresponding to the start position and the time corresponding to the end position during the movement of the user, to obtain the horizontal angle data.

10. The method according to claim 1, further comprising:

determining relative motion between the at least one measurement position based on the two-dimensional posture data corresponding to each posture signal.

11. A motion data calibration system, comprising:

at least one storage medium storing at least one instruction set for calibrating motion data; and

at least one processor in communication with the at least one storage medium, wherein

when the motion data calibration system runs, the at least one processor reads the at least one instruction set and implements the motion data calibration method according to any one of claims 1 to 10.

**100**

FIG. 1

<u>130</u>

| | | |
|---|---|---|
| ∿ 210 | ∿ 220 | ∿ 230 |
| Obtaining module | Processing module | Control module |
| ∿ 240 | ∿ 250 | ∿ 260 |
| Communic ations module | Power supply module | I/O module |

FIG. 2

<u>300</u>

380

370

360

350

From/to the network

Hard disk

I/O

Communications port

310

320

330

340

Processor

ROM

RAM

FIG. 3

**400**

FIG. 4

**500**

| Obtain an action signal during a movement of a user, wherein the action signal at least comprises myoelectric signal or a posture signal | 510 |

| Monitor the action of the movement of the user based on at least feature information corresponding to the myoelectric signal or feature information corresponding to the posture signal | 520 |

FIG. 5

**3000**

Obtain action data during a movement of a user, the action data comprises at least one posture signal corresponded to at least one measurement position on the user, each of the posture signal of the at least one posture signal comprises three-dimensional posture data under the original coordinate corresponded to the measurement position

S3020

Establish a target coordinate system

S3040

Convert each of the posture signal to two-dimensional posture data in the target coordinate system

S3060

Determine relative motion between the at least one measurement position based on the two-dimensional posture data corresponded to each of the posture signal

S3080

FIG. 6

FIG. 7

## S3060

Obtain a pre-stored conversion relationship between the target coordinate system and the original coordinate system      S3062

Convert each of the posture signal to three-dimensional motion data in the target coordinate system based on the conversion relationship between the target coordinate system and the original coordinate system      S3064

Convert the angular velocity data on the X-axis and the angular velocity data on the Y-axis to vertical angular velocity data by using a vector rule      S3066-2

S3066

Perform time integration on the vertical angular velocity data based on a time corresponded to a start position and an end position during movement of the user, to obtain the vertical angle data      S3066-4

Use the angular velocity data on the Z-axis as the horizontal angular velocity data      S3066-6

Perform time integration on the horizontal angular velocity data based on the time corresponded to the start position and the end position during movement of the user, to obtain the horizontal angle data      S3066-8

FIG. 8

FIG. 9

## INTERNATIONAL SEARCH REPORT

| | |
|---|---|
| | International application No. |
| | **PCT/CN2021/134421** |

**A.    CLASSIFICATION OF SUBJECT MATTER**

G06K 9/00(2022.01)i;  G06T 17/00(2006.01)i

According to International Patent Classification (IPC) or to both national classification and IPC

**B.    FIELDS SEARCHED**

Minimum documentation searched (classification system followed by classification symbols)

G06K; G06T

Documentation searched other than minimum documentation to the extent that such documents are included in the fields searched

Electronic data base consulted during the international search (name of data base and, where practicable, search terms used)

CNABS, CNTXT, VEN, USTXT, EPTXT, WOTXT, CNKI, IEEE: 运动, 三维, 二维, 姿态, 坐标, 监测, fitness, 3D, 2D, pose, coordinate, monitor

**C.    DOCUMENTS CONSIDERED TO BE RELEVANT**

| Category* | Citation of document, with indication, where appropriate, of the relevant passages | Relevant to claim No. |
|---|---|---|
| X | CN 110517336 A (BEIJING INSTITUTE OF TECHNOLOGY) 29 November 2019 (2019-11-29)<br>    description, paragraphs [0035]-[0064], and figures 1-5 | 1-11 |
| X | CN 110705496 A (CHENGDU CODOON INFORMATION TECHNOLOGY CO., LTD.) 17 January 2020 (2020-01-17)<br>    description, paragraphs [0012]-[0042], and figure 1 | 1-11 |
| A | CN 108939512 A (DALIAN UNIVERSITY OF TECHNOLOGY) 07 December 2018 (2018-12-07)<br>    entire document | 1-11 |
| A | CN 111353941 A (GUANGZHOU HUANTEK TECHNOLOGY CO., LTD. et al.) 30 June 2020 (2020-06-30)<br>    entire document | 1-11 |
| A | CN 111767932 A (BEIJING DEEP BLUE CHANGSHENG TECHNOLOGY CO., LTD.) 13 October 2020 (2020-10-13)<br>    entire document | 1-11 |

☑ Further documents are listed in the continuation of Box C.       ☑ See patent family annex.

| | |
|---|---|
| *       Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| **07 July 2022** | **27 July 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2021/134421** |

### C. DOCUMENTS CONSIDERED TO BE RELEVANT

| Category* | Citation of document, with indication, where appropriate, of the relevant passages | Relevant to claim No. |
| --- | --- | --- |
| A | JP 2013242790 A (NIPPON TELEGRAPH & TELEPHONE CORP.) 05 December 2013 (2013-12-05) entire document | 1-11 |
| A | US 2015294597 A1 (NEW YORK UNIVERSITY) 15 October 2015 (2015-10-15) entire document | 1-11 |

Form PCT/ISA/210 (second sheet) (January 2015)

**INTERNATIONAL SEARCH REPORT**
Information on patent family members

International application No.

**PCT/CN2021/134421**

| Patent document cited in search report | | | Publication date (day/month/year) | Patent family member(s) | | | Publication date (day/month/year) |
|---|---|---|---|---|---|---|---|
| CN | 110517336 | A | 29 November 2019 | CN | 110517336 | B | 05 February 2021 |
| CN | 110705496 | A | 17 January 2020 | None | | | |
| CN | 108939512 | A | 07 December 2018 | CN | 108939512 | B | 19 May 2020 |
| CN | 111353941 | A | 30 June 2020 | None | | | |
| CN | 111767932 | A | 13 October 2020 | None | | | |
| JP | 2013242790 | A | 05 December 2013 | JP | 5735455 | B2 | 17 June 2015 |
| US | 2015294597 | A1 | 15 October 2015 | WO | 2014066516 | A1 | 01 May 2014 |
| | | | | EP | 2912644 | A1 | 02 September 2015 |
| | | | | US | 9646514 | B2 | 09 May 2017 |

Form PCT/ISA/210 (patent family annex) (January 2015)